# EUROPEAN PATENT SPECIFICATION

(11) **EP 4 215 060 B1**
(45) Date of publication and mention of the grant of the patent: **25.12.2024**
(21) Application number: 23152483.6
(22) Date of filing: 19.01.2023
(51) Int. Cl.: A61K 31/295, A61K 47/38, A61P 7/06, A61K 9/70, A61K 47/36, A23L 33/10, A23L 33/125, A23L 33/24, A61K 33/26

(54) **PROCESS FOR PREPARATION OF A CONTROLLED-RELEASE SYSTEM**
PROZESS FÜR DIE ZUBEREITUNG EINES SYSTEMS ZUR KONTROLLIERTEN WIRKSTOFFFREISETZUNG
PROCÉDÉ DE PRÉPARATION D'UN SYSTÈME A LIBÉRATION RETARDÉE

(30) Priority: 20.01.2022 IT 202200000953
(43) Date of publication of application: 26.07.2023
(73) Proprietor: BIONATIVA S.P.A., 50028 Barberino Tavarnelle (FI) (IT)
(72) Inventor: ANZAGHI, Piergiorgio, 20078 SAN COLOMBANO AL LAMBRO (MI) (IT)
(74) Representative: Firmati, Leonardo

(56) References cited:
- WO-A1-2016/125077
- CN-A- 112 274 633
- DE-U1- 202021 104 247
- JP-A- 2019 202 963

## Description

### Field of invention

The present invention relates to a process for the preparation of a system for the controlled release of a biologically active substance, wherein said system is based on hyaluronic acid. The invention also relates to the system obtained by said process, and to said system for use as a medicament or for use in the treatment of iron deficiency or iron-deficiency anaemia.

### Prior art

Hyaluronic acid (HA) is a heteropolysaccharide (glycosaminoglycan) consisting of alternating residues of D-glucuronic acid and N-acetyl-D-glucosamine.

Hyaluronic acid is one of the fundamental ingredients of the connective tissues of humans and other mammals. It is found in all the connective tissues of the human body, for example in skin, cartilage and tendons.

Hyaluronic acid strengthens tissues and joints, and is an important constituent of joint fluid, wherein it acts as a lubricant.

Hyaluronic acid also plays a crucial role in tissue-repair processes.

Hyaluronic acid is used in cosmetic preparations, for example as a subcutaneously injected wrinkle filler, and in pharmaceutical preparations.

Iron is an essential mineral for some biological functions, including haemoglobin formation. Iron deficiency, caused by a low dietary intake, absorption problems or blood losses, gives rise to insufficient haemoglobin production, which causes low oxygen circulation in the body.

Iron-deficiency anaemia is the most common form of anaemia. It is also called sideropenic anaemia (from the Greek síderos = iron and penía = poverty, lack). It arises when the body's iron levels are inadequate.

Iron resources can be maintained and reconstituted by administering iron-based formulations.

Said formulations can contain elemental iron, in ferric or ferrous form. Iron in ferrous form is mainly used, because it guarantees better absorption. Ferrous iron is found, for example, in the form of ferrous sulphate, ferrous fumarate and ferrous gluconate.

Slow-release formulations release iron slowly during the intestinal transit of the product, thereby improving its absorption and tolerability.

WO 2016/125077 A1 discloses slow release compositions based on alginate, cellulose derivatives and mannose for the delivery of active agents, such as iron salts, as well as a process for their preparation.

Oral treatment often causes gastrointestinal side effects, such as heartburn, abdominal pains, nausea and constipation.

There is consequently a need to prepare new and improved modified-release iron delivery systems.

### Brief description of figures

Figure 1 shows the SEM photo relating to Sample 1.
Figure 2 shows an expansion of Figure 1 highlighting the three-dimensional structures.

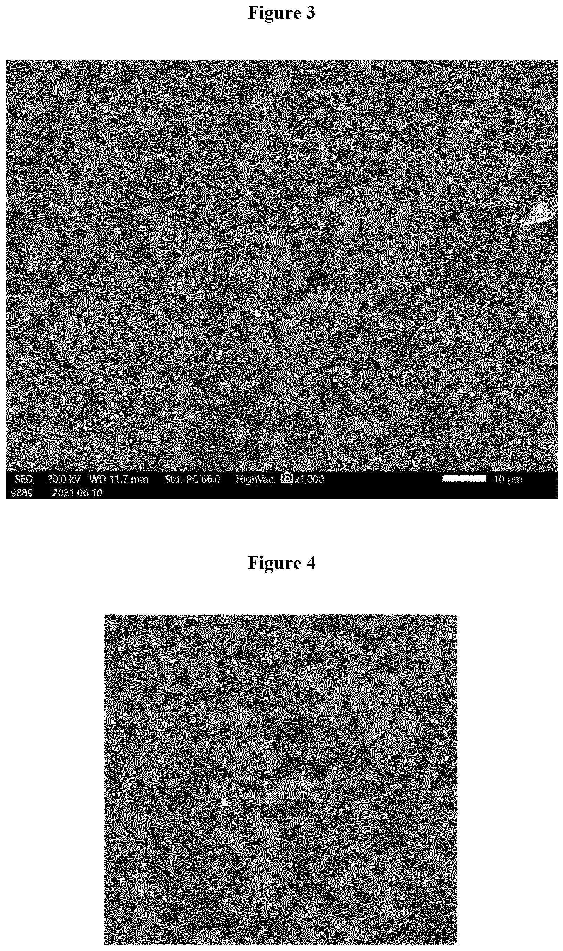

Figure 3 shows the SEM photo relating to Sample 2.
Figure 4 shows an expansion of Figure 3 highlighting the three-dimensional structures.

### Summary of the invention

The object of the present invention is a process for the preparation of a system for the controlled release of a biologically active substance comprising the following steps:
a) hydrolysis in acid medium of hyaluronic acid, or a salt thereof, at a pH ranging between 2 and 5;
b) addition of a cellulose derivative to the hydrolysate obtained in step a);
c) addition of a biologically active substance;
d) addition of a first gelling agent;
e) addition of mannose, optionally in combination with a second gelling agent; wherein

the biologically active substance is an iron salt, preferably organic; and
the first gelling agent is selected from the group consisting of calcium ions (Ca²⁺) or magnesium ions (Mg²⁺) or combinations thereof.

A further object of the present invention is a controlled-release system of a biologically active substance obtained by the preparation process described above, and said controlled release system for use as defined in claims 16 or 17.

### Detailed description of the invention

The object of the present invention is a process for the preparation of a system for the controlled or modified release of a biologically active substance comprising the following steps:
a) hydrolysis in acid medium at a pH ranging between 2 and 5 of hyaluronic acid, or a salt thereof, preferably in the presence of mannose.
b) addition of a cellulose derivative to the hydrolysate obtained in step a);
c) addition of a biologically active substance;
d) addition of a first gelling agent;
e) addition of mannose, optionally in combination with a second gelling agent, preferably with hydroxyethylcellulose;
wherein
the biologically active substance added in step c) is an iron salt, preferably organic; and
the first gelling agent is selected from the group consisting of calcium ions (Ca²⁺) or magnesium ions (Mg²⁺) or combinations thereof.

The process according to the invention can optionally comprise a stabilisation step f) involving the addition of one or more rheology modifiers selected from the group comprising or consisting of a polysaccharide, preferably inulin, cellulose or derivatives thereof, such as hydroxyethylcellulose, optionally combined with mannose. In the context of the present invention, the expression "rheology modifiers" means substances able to modify the density and/or viscosity of the controlled- or modified-release system.

A base, such as NaOH, is preferably added to the mixture obtained at the end of step b), to reach a pH ranging between 6 and 8, preferably between 6 and 7, and mannose is optionally added before step c).

According to the invention, the expression "in acid medium" means that the hydrolysis is conducted at a pH ranging between 2 and 5, preferably between 3 and 4, and more preferably equal to 3.7.

The hydrolysis is conducted in the presence of at least one inorganic or organic acid, preferably a polyprotic acid, and preferably an inorganic acid. The acid can be selected from phosphoric acid, lactic acid, citric acid, tartaric acid or combinations thereof; it is preferably selected from phosphoric acid and lactic acid, and is most preferably phosphoric acid.

According to a preferred aspect of the invention, the hydrolysis step is conducted at a temperature ranging between 12 and 70°C, more preferably between 37 and 65°C, for a time ranging between 1 h and 78 h, more preferably between 10 h and 26 h.

The hyaluronic acid of step a) before hydrolysis has an average MW (molecular weight) preferably ranging between 500 and 2,000 kDa, more preferably between 800 and 1,800 kDa; and even more preferably between 1,300 and 1,500 kDa. According to a further preferred aspect of the invention, the hyaluronic acid according to the present invention has an average MW of about 1,400 kDa.

According to the present invention, the (average) molecular weight of hyaluronic acid is calculated by the gel permeation chromatography (GPC) analysis method.

The hyaluronic acid is preferably in the form of sodium salt.

The hyaluronic acid, preferably in the form of sodium salt, is used in amounts ranging between 0.1 and 1.0wt% of the total weight of the controlled- or modified-release system, preferably ranging between 0.2 and 0.5wt% of the total weight of the controlled- or modified-release system, and more preferably amounts to 0.3wt% of the total weight of the controlled- or modified-release system.

According to the present invention, the percentage by weight (wt%) is based on the total weight of the controlled- or modified-release system obtainable by the process according to the invention.

The cellulose derivative can be selected from hydroxyethylcellulose, methylethylcellulose, methylcellulose, propylcellulose or combinations thereof; preferably hydroxyethylcellulose.

The biologically active substance is an iron salt, preferably organic; the iron salt is preferably selected from iron bisglycinate and iron fumarate; the iron salt is preferably iron bisglycinate.

The biologically active substance can be used in amounts ranging between 0.1 and 1.0wt% of the total weight of the controlled- or modified-release system, preferably between 0.2 and 0.5wt% of the total weight of the controlled- or modified-release system, and more preferably amounts to 0.3wt% of the total weight of the controlled- or modified-release system.

The first gelling agent can be selected from calcium ions (Ca²⁺) or magnesium ions (Mg²⁺) or combinations thereof, and is preferably represented by calcium ions (Ca²⁺).

The second gelling agent can be represented by calcium ions (Ca²⁺).

A further object of the present invention is a system for the controlled or modified release of a biologically active substance obtained by the preparation process described above.

The controlled- or modified-release systems according to the invention can be used to treat iron deficiencies and iron-deficiency anaemia.

The systems according to the invention advantageously enable the amount of iron incorporated in the three-dimensional "box" system obtained by the process according to the invention to be increased, and allow controlled, modified release during gastrointestinal transit, thus giving rise to therapeutic efficacy, resulting in better patient compliance with the treatment.

The examples below further illustrate the invention.

### Examples

### Example 1

In a beaker, 530 mg of sodium hyaluronate was added to 200 mL of distilled water at 50°C, and left under stirring for 3 h until completely dissolved. 40 mg of mannose and 200 µL of phosphoric acid were then added, and the mixture was left under stirring for 1.5 h. 236 mg of hydroxyethylcellulose was then added, and the mixture was left under stirring for 24 h at 50°C.

After 24 h a solution of 1M NaOH (≈ 4 mL) was added until pH 6-7 was reached. The reaction mixture was cooled to room temperature (≈ 25°C), and 16 mg of mannose was added. After 15 minutes, 600 mg of Fe bisglycinate was added, and the solution was left under stirring for 1 h. 120 µL of an 18 mM aqueous solution of CaCl₂ and 68 µL of an 18 mM aqueous solution of MgCl₂ were then added, followed by 322 mg of hydroxyethylcellulose and 272 mg of mannose. The stirring direction was then reversed, and 200 µL of a saturated aqueous solution of Ca(OH)₂ was added.

### Example 2 - (comparative example)

In a beaker, 530 mg of alginic acid was added to 200 mL of distilled water at 50°C, and left under stirring for 3 h until completely dissolved. 40 mg of mannose and 200 µL of phosphoric acid were then added, and the mixture was left under stirring for 1.5 h. 236 mg of hydroxyethylcellulose was then added, and the mixture was left under stirring for 24 h at 50°C.

After 24 h a solution of 1M NaOH (≈ 4 mL) was added until pH 6-7 was reached. The reaction mixture was cooled to room temperature (≈ 25°C), and 16 mg of mannose was added. After 15 minutes, 600 mg of Fe bisglycinate was added, and the solution was left under stirring for 1 h. 120 µL of an 18 mM aqueous solution of CaCl₂ and 68 µL of an 18 mM aqueous solution of MgCl₂ were then added, followed by 322 mg of hydroxyethylcellulose and 272 mg of mannose. The stirring direction was then reversed, and 200 µL of a saturated aqueous solution of Ca(OH)₂ was added.

### Example 3

Two samples were prepared:
1. Sample with sodium hyaluronate prepared according to Example 1: Sample 1.
2. Sample with alginic acid prepared according to Example 2: Sample 2.

Both samples were analysed by SEM to evaluate their morphology, and by EDS to evaluate their composition along the surface, with particular reference to iron distribution.

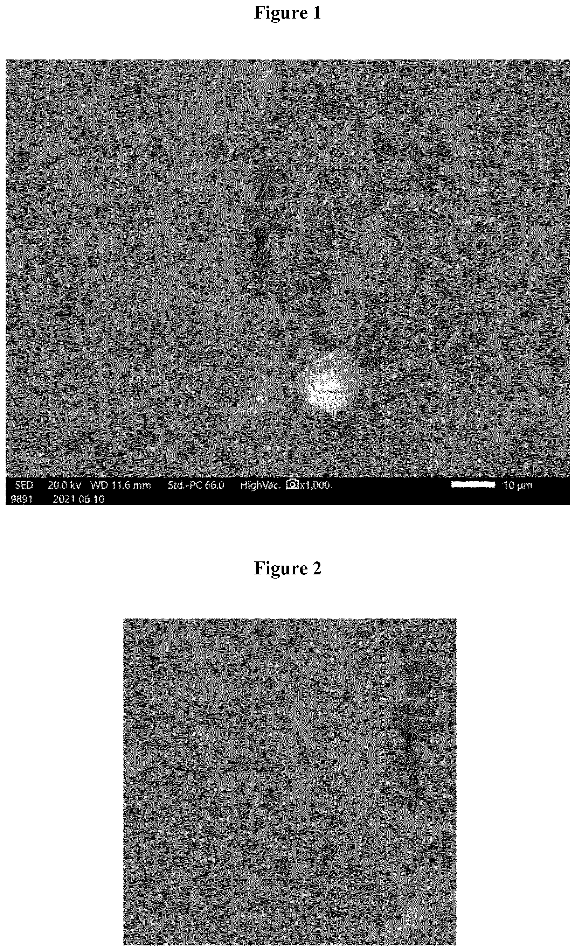

Figure 1 is the SEM photo relating to Sample 1.

Figure 2 is the SEM photo relating to Sample 2.

The samples show a non-uniform surface, with differently-coloured areas.

The samples were then analysed by EDS, and in particular, the chemical composition of the two zones, light and dark, was compared. The results shown in Table 1 relate to the mass percentage of carbon, oxygen and iron in the two areas analysed for each sample.

**Table 1**

| *Sample* | *Area* | *C (mass%)* | *O (mass%)* | *Fe (mass%)* |
|---|---|---|---|---|
| **Sample 1** | Light | 24.51±0.12 | 25.06±0.14 | 18.27±0.11 |
| | Dark | 33.90±0.11 | 22.60±0.13 | 7.83±0.06 |
| **Sample 2** | Light | 19.02±0.08 | 32.24±0.12 | 7.84±0.06 |
| | Dark | 24.10±0.09 | 28.33±0.13 | 2.97±0.04 |

Sample 1 exhibited an iron concentration along the surface, with a higher concentration in the lightest zones and a higher concentration in general than that of sample 2 in both the light and dark zones.

This demonstrates that the controlled-release system based on the sodium hyaluronate according to the invention stores iron more effectively.

## Claims

1. A process for the preparation of a controlled- or modified-release system of a biologically active substance, comprising the following steps:
a) hydrolysis of hyaluronic acid, or a salt thereof, in an acid medium, at a pH ranging between 2 and 5;
b) addition of a cellulose derivative to the hydrolysate obtained in step a);
c) addition of a biologically active substance;
d) addition of a first gelling agent;
e) addition of mannose, optionally in combination with a second gelling agent,
wherein
the biologically active substance is an iron salt, preferably organic; and
the first gelling agent is selected from the group consisting of calcium ions (Ca²⁺) or magnesium ions (Mg²⁺) or combinations thereof.

2. Process according to claim 1, wherein in step a) the hydrolysis is conducted in the presence of mannose.

3. Process according to claim 1 or 2, wherein step e) is conducted in the presence of hydroxyethylcellulose.

4. Process according to claims 1-3, further comprising a stabilisation step f) of addition of one or more rheology modifiers, selected from the group comprising a polysaccharide, preferably inulin, cellulose or derivatives thereof, optionally in combination with mannose.

5. Process according to claims 1-4, wherein a base is added to the mixture obtained at the end of step b) to reach a pH ranging between 6 and 8, preferably between 6 and 7, and mannose is optionally added, before step c).

6. Process according to claims 1-5, wherein the hydrolysis is carried out at a pH ranging between 3 and 4, preferably equal to 3.7.

7. Process according to claims 1-6, wherein the hydrolysis is carried out in the presence of at least one acid selected from phosphoric acid, lactic acid, citric acid, tartaric acid or combinations thereof; preferably from phosphoric acid and lactic acid, more preferably is phosphoric acid.

8. Process according to claims 1-7, wherein the hyaluronic acid before hydrolysis has an average MW ranging between 500 and 2,000 kDa, preferably between 800 and 1,800 kDa; more preferably between 1,300 and 1,500 kDa; most preferably equal to 1,400 kDa.

9. Process according to claims 1-8, wherein the hyaluronic acid is in the form of a sodium salt.

10. Process according to claims 1-9, wherein the hyaluronic acid, or the sodium salt thereof, is used in an amount ranging between 0.1 and 1.0 wt% of the total weight of the controlled- or modified-release system, preferably ranging between 0.2 and 0.5 wt%, and more preferably equal to 0.3 wt%.

11. Process according to claims 1-10, wherein the cellulose derivative is selected from hydroxyethylcellulose, methylethylcellulose, methylcellulose and propylcellulose or combinations thereof; preferably is hydroxyethylcellulose.

12. Process according to claims 1-11, wherein the biologically active substance is selected from iron bisglycinate and iron fumarate; and more preferably is iron bisglycinate.

13. Process according to claims 1-12, wherein the biologically active substance is used in an amount ranging between 0.1 and 1.0 wt% on the total weight of the controlled- or modified-release system, preferably between 0.2 and 0.5 wt%, more preferably equal to 0.3 wt%.

14. Process according to claims 1-13, wherein the second gelling agent is calcium ions (Ca²⁺).

15. Controlled- or modified-release system of a biologically active substance obtainable by the process according to claims 1-14.

16. Controlled- or modified-release system according to claim 15, for use as a medicament.

17. Controlled- or modified-release system according to claim 16, for use in the treatment of iron deficiency or iron-deficiency anaemia.

## Patentansprüche

1. Verfahren zur Herstellung eines Systems mit kontrollierter oder modifizierter Freisetzung einer biologisch aktiven Substanz, umfassend die folgenden Schritte:
a) Hydrolyse von Hyaluronsäure oder einem Salz davon in einem sauren Medium bei einem pH-Wert im Bereich zwischen 2 und 5;
b) Zugabe eines Cellulosederivats zu dem in Schritt
a) erhaltenen Hydrolysat;
c) Zugabe einer biologisch aktiven Substanz;
d) Zugabe eines ersten Geliermittels;
e) Zugabe von Mannose, gegebenenfalls in Kombination mit einem zweiten Geliermittel, wobei die biologisch aktive Substanz ein Eisensalz, vorzugsweise organisch, ist; und das erste Geliermittel ist aus der Gruppe bestehend aus Calciumionen (Ca²⁺) oder Magnesiumionen (Mg²⁺) oder Kombinationen davon ausgewählt.

2. Verfahren nach Anspruch 1, wobei in Schritt a) die Hydrolyse in Gegenwart von Mannose durchgeführt wird.

3. Verfahren nach Anspruch 1 oder 2, wobei Schritt e) in Gegenwart von Hydroxyethylcellulose durchgeführt wird.

4. Verfahren nach den Ansprüchen 1-3, ferner umfassend einen Stabilisierungsschritt f) der Zugabe eines oder mehrerer Rheologie-Modifikatoren, ausgewählt aus der Gruppe, umfassend ein Polysaccharid, vorzugsweise Inulin, Cellulose oder Derivate davon, gegebenenfalls in Kombination mit Mannose.

5. Verfahren nach den Ansprüchen 1-4, wobei der am Ende von Schritt b) erhaltenen Mischung eine Base zugesetzt wird, um einen pH-Wert im Bereich zwischen 6 und 8, vorzugsweise zwischen 6 und 7, zu erreichen, und gegebenenfalls vor Schritt c) Mannose zugegeben wird.

6. Verfahren nach den Ansprüchen 1-5, wobei die Hydrolyse bei einem pH-Wert im Bereich zwischen 3 und 4, vorzugsweise gleich 3,7, durchgeführt wird.

7. Verfahren nach den Ansprüchen 1-6, wobei die Hydrolyse in Gegenwart von mindestens einer Säure durchgeführt wird, die aus Phosphorsäure, Milchsäure, Zitronensäure, Weinsäure oder Kombinationen davon ausgewählt ist; vorzugsweise aus Phosphorsäure und Milchsäure, bevorzugter ist Phosphorsäure.

8. Verfahren nach den Ansprüchen 1-7, wobei die Hyaluronsäure vor der Hydrolyse ein durchschnittliches Molekulargewicht im Bereich zwischen 500 und 2.000 kDa, vorzugsweise zwischen 800 und 1.800 kDa; bevorzugter zwischen 1.300 und 1.500 kDa; am bevorzugtesten gleich 1.400 kDa aufweist.

9. Verfahren nach den Ansprüchen 1-8, wobei die Hyaluronsäure in Form eines Natriumsalzes vorliegt.

10. Verfahren nach den Ansprüchen 1-9, wobei die Hyaluronsäure oder das Natriumsalz davon in einer Menge im Bereich zwischen 0,1 und 1,0 Gew.-% des Gesamtgewichts des Systems mit kontrollierter oder modifizierter Freisetzung, vorzugsweise im Bereich zwischen 0,2 und 0,5 Gew.-% und bevorzugter gleich 0,3 Gew.-% verwendet wird.

11. Verfahren nach den Ansprüchen 1-10, wobei das Cellulosederivat aus Hydroxyethylcellulose, Methylethylcellulose, Methylcellulose und Propylcellulose oder Kombinationen davon ausgewählt ist; vorzugsweise ist es Hydroxyethylcellulose.

12. Verfahren nach den Ansprüchen 1-11, wobei die biologisch aktive Substanz aus Eisenbisglycinat und Eisenfumarat ausgewählt ist; und bevorzugter ist sie Eisenbisglycinat.

13. Verfahren nach den Ansprüchen 1-12, wobei die biologisch aktive Substanz in einer Menge im Bereich zwischen 0,1 und 1,0 Gew.-%, bezogen auf das Gesamtgewicht des Systems mit kontrollierter oder modifizierter Freisetzung, vorzugsweise zwischen 0,2 und 0,5 Gew.-%, bevorzugter gleich 0,3 Gew.-%, verwendet wird.

14. Verfahren nach den Ansprüchen 1-13, wobei das zweite Geliermittel Calciumionen (Ca²⁺) ist.

15. Systems mit kontrollierter oder modifizierter Freisetzung einer biologisch aktiven Substanz, die durch das Verfahren nach den Ansprüchen 1-14 erhältlich ist.

16. System mit kontrollierter oder modifizierter Freisetzung nach Anspruch 15 zur Verwendung als Medikament.

17. System mit kontrollierter oder modifizierter Freisetzung nach Anspruch 16 zur Verwendung bei der Behandlung von Eisenmangel oder Eisenmangelanämie.

## Revendications

1. Procédé de préparation d'un système à libération contrôlée ou modifiée d'une substance biologiquement active, comprenant les étapes suivantes :
a) hydrolyse de l'acide hyaluronique, ou d'un de ses sels, dans un milieu acide, à un pH compris entre 2 et 5 ;
b) ajout d'un dérivé de cellulose à l'hydrolysat obtenu à l'étape a) ;
c) ajout d'une substance biologiquement active ;
d) ajout d'un premier agent gélifiant ;
e) ajout de mannose, éventuellement en combinaison avec un second agent gélifiant, dans lequel la substance biologiquement active est un sel de fer, de préférence organique, et le premier agent gélifiant est choisi dans le groupe constitué par les ions calcium (Ca²⁺) ou les ions magnésium (Mg²⁺) ou des combinaisons de ceux-ci.

2. Procédé selon la revendication 1, dans lequel, à l'étape a), l'hydrolyse est effectuée en présence de mannose.

3. Procédé selon la revendication 1 ou 2, dans lequel l'étape e) est réalisée en présence d'hydroxyéthylcellulose.

4. Procédé selon les revendications 1-3, comprenant de plus une étape de stabilisation f) consistant à ajouter un ou plusieurs modificateurs de rhéologie, choisis dans le groupe comprenant un polysaccharide, de préférence l'inuline, la cellulose ou leurs dérivés, éventuellement en combinaison avec le mannose.

5. Procédé selon les revendications 1-4, dans lequel une base est ajoutée au mélange obtenu à la fin de l'étape b) pour atteindre un pH compris entre 6 et 8, de préférence entre 6 et 7, et du mannose est éventuellement ajouté, avant l'étape c).

6. Procédé selon les revendications 1-5, dans lequel l'hydrolyse est réalisée à un pH compris entre 3 et 4, de préférence égal à 3,7.

7. Procédé selon les revendications 1-6, dans lequel l'hydrolyse est réalisée en présence d'au moins un acide choisi parmi l'acide phosphorique, l'acide lactique, l'acide citrique, l'acide tartrique ou des combinaisons de ceux-ci ; de préférence parmi l'acide phosphorique et l'acide lactique, de préférence encore l'acide phosphorique.

8. Procédé selon les revendications 1-7, dans lequel l'acide hyaluronique avant hydrolyse a un poids moléculaire moyen compris entre 500 et 2 000 kDa, de préférence entre 800 et 1 800 kDa ; plus préférablement entre 1 300 et 1 500 kDa ; idéalement égal à 1 400 kDa.

9. Procédé selon les revendications 1-8, dans lequel l'acide hyaluronique se présente sous la forme d'un sel de sodium.

10. Procédé selon les revendications 1-9, dans lequel l'acide hyaluronique, ou son sel de sodium, est utilisé dans une quantité comprise entre 0,1 et 1,0 % en poids du poids total du système à libération contrôlée ou modifiée, de préférence entre 0,2 et 0,5 % en poids, et plus préférablement égale à 0,3 % en poids.

11. Procédé selon les revendications 1-10, dans lequel le dérivé de cellulose est choisi parmi l'hydroxyéthylcellulose, la méthyléthylcellulose, la méthylcellulose et la propylcellulose ou leurs combinaisons ; de préférence, il s'agit de l'hydroxyéthylcellulose.

12. Procédé selon les revendications 1-11, dans lequel la substance biologiquement active est choisie parmi le bisglycinate de fer et le fumarate de fer ; de préférence, il s'agit du bisglycinate de fer.

13. Procédé selon les revendications 1-12, dans lequel la substance biologiquement active est utilisée dans une quantité comprise entre 0,1 et 1,0 % en poids du poids total du système à libération contrôlée ou modifiée, de préférence entre 0,2 et 0,5 % en poids, plus préférablement égale à 0,3 % en poids.

14. Procédé selon les revendications 1-13, dans lequel le second agent gélifiant est un ion calcium (Ca²⁺) .

15. Système à libération contrôlée ou modifiée d'une substance biologiquement active susceptible d'être obtenu par le procédé selon les revendications 1-14.

16. Système à libération contrôlée ou modifiée selon la revendication 15, pour une utilisation comme médicament.

17. Système à libération contrôlée ou modifiée selon la revendication 16, pour une utilisation dans le traitement de la carence en fer ou de l'anémie ferriprive.
